# EUROPEAN PATENT APPLICATION

(11) **EP 2 612 667 A2**
(43) Date of publication of application: **10.07.2013**
(21) Application number: 11821203.4
(22) Date of filing: 29.08.2011
(51) Int. Cl.: A61K 31/235, A61K 35/64, A61P 1/02

(54) **ANTICARIES DENTIFRICE COMPOSITION THAT INCLUDES ANTICARIES PROPOLIS AS AN ACTIVE PRINCIPLE**

(30) Priority: 31.08.2010 CL 9312010
(71) Applicant: Universidad De La Frontera, Temuco 4811230 (CL); Pontificia Universidad Católica De Chile, 8331150 - Santiago (CL); Knop Laboratorios Ltda, Quilpué 2420548 (CL)
(72) Inventor: SALAZAR NAVARRETE, Luis, Antonio, Temuco 4811230 (CL); MONTENEGRO RIZZARDINNI, Gloria, Del Carmen, Santiago 8331150 (CL)
(74) Representative: Carvajal y Urquijo, Isabel
(86) International application number: PCT/IB2011/053778
(87) International publication number: WO 2012/029015

(57) **Abstract**

The invention is referred to a new dentifrice composition with anticariogenic properties, which has as active anticariogenic ingredients a combination of propolis, methylparaben and propylparaben. From these 3 components, propolis is the major component, which is reinforced in its activity for a smaller amount of methylparaben and propylparaben obtaining a composition with a high anticariogenic composition without side effects. These active ingredients are present in the following percentages in relation to the total weight of the dentifrice composition:
2 a 10 % of propolis;
0,01 a 0,30% of methylparaben
0,01 a 0,15% of propylparaben

The dentifrice composition also includes a fluorinated compound and other auxiliary components usually used for formulation such as abrasives, moisturizing, foaming agents, thickeners, sweeteners, flavorants and demineralized water.

## Description

### Field of invention

The invention is focused on a dentifrice composition, specifically, in toothpaste for massive use, which has a natural active ingredient, specifically propolis. The use of this anticariogenic dentifrice composition decreases the bacterial load of *Streptococcus,* from the mutants group, with a 70% of effectiveness without side effects. Therefore, this dentifrice may be used for long time (>1 month) and could be purchased over the counter without doctor's prescription.

### Background of the invention

Cavities are one of the more prevalent diseases that affect to a large group of people worldwide, not matter the sex, age or social class. Currently in developed countries, this disease affects to the scholar and adult populations in a percentage ranging from 60 to 90%. Although, according to some studies, the incidence of this disease in the world population is approximately 94%, in Chile, the situation is not very different, because cavities are the oral pathology more prevalent, which affect around 90% of the population.

It is also necessary to consider that if this disease is not treated on time the lost of the tooth may occur, which is associated to other functional complications, such as, loss of masticatory ability and aesthetic and psychological problems, which involves economic and social costs.

The increase of bacterial resistance to anti-microbial agents, above all the synthetic ones, and the substantial increases on demand for natural products urges the necessity for new dentifrice formulations with anticariogenic properties as in the case of this invention.

### Ethiology of cavities

Cavities are an infectious pathological process, multifactorial and transmissible process that progressively destroys hard tissues, which may cause the tooth loss when is not treated on time. This disease is also associated to other functional complications, such as, loss of masticatory ability and aesthetic and psychological problems, which involve economic and social costs. Cavities involve the interaction between a causal agent (cariogenic bacterium), a substrate, where the bacterium lives and cause disease (dietary sugar), and other group of factors related to the host defenses (saliva, teeth). When one of these factors is missed cavities do not occur. Teeth susceptibility is given for a shortage of protective fluoride, which is found in the dental enamel or because some damage or fissure produced by bacterial plaque.

It is known that a large amount of bacteria that co-exist into the oral cavity (> 300 species), mostly non-pathogenic; therefore, -cavities are considered as a consequence of an oral ecosystem imbalance, which lead to the predominance of pathogens over the normal bacterial flora of the oral cavity. Among the bacteria species found into the mouth, just a certain group of them are able to produce acids that cause cavities. The main microorganisms associated to the production of cavities are, in order of frequency: *Streptococcus mutans* (mainly serotype c), and in less proportion S. *sobrinus, S. gordonii*, and species of *Lactobacillus* and *Actinomycetes.* There are also other species that belong to de genus *Streptococci,* such as, *S. salivarius, S. sanguinis* and *S*. *downei.* Dental plaque is a colorless biofilm that adheres to the teeth and gums hosting bacteria that produce acids. This plaque maintains the organics products produced during the bacterial metabolisms, which are in close contact with the enamel surface. The effect of organic acids as lactic acid, which is elaborated for plaque microorganisms, causes tooth demineralization and lastly cavities. However, to achieve concentrations of acids that could be destructive enough, it is necessary a substantial accumulation of acidogenic bacteria in the plaque. The accumulation process is started by the extracellular enzyme call glucosyltransferase (GTF), which synthesizes insoluble glycogen from sucrose molecules. This process allows the cariogenic bacteria be irreversibly attached to the tooth surface. When there is not food in the mouth the plaque pH remains constant; however, once the food is ingested, the pH decreases (pH<5.5 is considered critic), and the acid starts to dissolve the dental enamel. This process continues for 20 to 30 minutes until the saliva neutralizes the acidity of plaque, restoring the pH, and conducting the remineralization of the dental erosion. When the effect of the acids is frequent or continuous for a long period of time, the decalcification of the enamel is produced causing a fast dentin demineralization and degradation. Therefore, the acidogenic (ability to produce acids) and aciduric properties (ability to survive in acidic media) all together with the ability of synthesizes glucans, are the more important factors required for establishment and development of cariogenic biofilms.

### Prevention strategies.

The diet quality is a factor highly related with the formation of cavities. The number of bacteria and their degree of virulence is highly associated to the intake of sugar; therefore it is recommended to reduce the consumption of sugar to reduce cavities. Most experts agree that a good oral prevention considers a toothbrushing before and after each meal and flossing daily, which is considered enough to remove plaque and provide an effective control of surface cavities. In this way, there is in the market a variety of manual toothbrush and other a little bit more advanced in technology, either electric or ultrasonic. There is also a variety of toothpaste that mostly does not have antibacterial components in its formulation, and generally speaking, their active ingredients are fluoride, whose mode of action will be addressed later. Either controlling the use of sugar in the diet and a good oral hygiene are two strategies that involve a change in the population habits, which have been tried to implement without high rates of success.

A strategy that has been relatively successful is the use of fluorides. These compounds have a protective effect against cavities providing to the enamel a higher resistance against the acids that help to produce cavities. Fluorides may be incorporated to the drinking water, food or could be directly ingested as tablets or drops of sodium fluoride. These fluorides may also be applied topically for experts over the teeth. In the market there are toothpaste and mouthwash that are recommended not only for adults but also for kids, whose active ingredients are fluorides. However, the chronic ingestion of fluorides in excessive doses during the first years of life may produce enamel alterations, which are characterized for a chalky-white opacity that could eventually affect the complete dental surface, and in some severe cases, may affect the tooth shape, produce pits, cracks and brown spots.

Other technology used for control of cavities is the application of dental sealants. These are resins that are applied on the tooth covering cracks and fissures and protecting the teeth against bacterial contact. However, this procedure may be applied only for experts in healthy molars and teeth.

### Antibacterial therapies and their mode of action

In these therapies consider the use of agents that acts over the plaque, which removed the microorganisms that produce the plaque inhibiting the formation of the plaque matrix and removing the plaque already formed. Some of these agents may be applied topically for experts or be part of mouthwashes and toothpaste. Some of the more studied and used agents are:
Chlorhexidine (1:6-Di 4'-chlorophenyl-biguanide-dohexane): Belongs to the group of biguanide. Is the most widely used compound to control microorganisms that cause cavities. It acts increasing the permeability of the cell membrane and the coagulation of cytoplasmic macromolecules. Among its advantages could be mentioned: is effective preventing accumulation of plaque, has a wide spectrum and substantivity, ranging from 7 to 12 hours. Their disadvantages are: do not remove the plaque already formed, its flavor is unpleasant (metallic), staines teeth and mucous membrane, it is difficult to be incorporated in the toothpaste formulation, in some cases may produce desquamation of the oral mucosa, can also promote the formation of supragingival calculus, parotid swelling, and could be only used for short period of time.
CPC (cetylpyridinium chloride): This is a quaternary ammonium compound that in the long-term has a moderate inhibitory activity against plaque. This compound is absorve by the dental mucosa disabling its interaction with the bacteria. The formulations that contain this compound stain teeth. Listerine (Phenolic antiseptic): It is widely used in mouthwash formulations. It has a moderate effect on growing of plaque and some anti-inflamatory effect. It has a poor oral retention.
Hexetidine: Its substantivity goes from 1 to 3 hours. When is used in high concentrations it causes ulcerations. Its inhibitory effect over plaque is low when compared with clorhexidine.
Triclosan (trichloro-2'-hydroxydiphenyl ether): Its plaque inhibitory effect is moderated, however, does not significantly stains teeth. It reduces the gingival inflammation and its substantivity is up to 14 hours.
Delmopinol: Affects the bacterial adhesion to the teeth, inhibiting the formation of plaque and gingivitis. Among its more important advantages are: its effect over the microbial population, its potential effect as anesthetic, and the low staining of teeth. However, it substantivity lasts only 30 minutes.
Oxygenated agents: Similar to hydrogen peroxide, which mainly inhibits anaerobic bacteria.
Salifluor (5n-octanoyl-3'-trifluoromethylsalicylanide): This product controls plaque and gingivitis, but it is not known its mode of action.

Ultimately, the applied preventive programs have not been enough to reduce the prevalence of cavities around the world. A present, the anticariogenic active compounds have adverse side effects or are not effective enough. Is for this reason that a new dentifrice composition with good anticariogenic effectiveness and without side effects is needed. Moreover, the demand for natural products has been highly increasing. This is solved with the invention of this dentifrice composition, where the active ingredient is propolis, a natural compound that has a high anticariogenic activity and does not have side effects.

### Summary of the invention

The invention refers to a new dentifrice composition with anticariogenic properties, which has as anticariogenic active ingredient a combination of propolis, methylparaben and propylparabe. From these three components propolis is the major component, which is reinforced in its activity for a minor amount of methylparaben and propylparaben giving rise to a composition with a high anticariogenic activity without side effects. These active ingredients are present in the following percentages with reference to the total weight of the dentifrice composition:
2 to 10 % of propolis;
0,01 to 0,30% of methylparaben; and
0,01 to 0,15% of propylparaben;

Where the dentifrice composition also consider a fluorinated compound and other agents of formulation usually used, such as, abrasives, moisturizing, foaming agents, thickeners, sweeteners, flavorants and demineralized water.

### Detailed description of the invention

The invention refers to a new dentifrice composition preferably toothpaste, because its massive use. Its active ingredient is a natural product that reduces the bacterial load of *Streptococus* from the mutants group. It is expected that this product reaches at least 70% of effectiveness, that can be used for a long period of time, does not causes side effects, and therefore, could be purchased over the counter without doctor's prescription.

This new dentifrice composition with anticariogenic properties has as anticariogenic active ingredient a combination of propolis, methylparaben and propylparaben. From these three components propolis is the major ingredient, which is reinforced in its activity for a minor amount of methylparaben and propylparaben. This combination gives rise to a final composition characterized for its high level of anticariogenic activity without side effects. These active ingredients are in the following percentages with respect to the total weight of the dentifrice composition:
2 to10 % of propolis extracts;
0,01 to 0,30% of methylparaben; and
0,01 to 0,15% of propylparaben;
where the dentifrice composition has additionally a fluorinated compound and other agents of formulation usually used, such as, abrasives, moisturizing, foaming agents, thickeners, sweeteners, flavorants and demineralized water.

Propolis is the main anticariogenic active ingredient of this invention. Propolis is a brownish resinous substance, non-toxic, which is collected by bees, and it is used in the hive as glue, insulating and antiseptic. Has been observed that resinous compounds that are present in the crude propolis are originated from three sources: plant exudates collected by bees, substances produced as result of bees' metabolism, other material incorporated during propolis elaboration.

Propolis composition is variable according to the plants species established in the area where the bees are present. Moreover, bees make a selective use of vegetables resources available around the apiary.

In general, crude propolis are composed for approximately an 50% of resin and vegetal balm, 30% of wax, 10% of essential aromatic oils, 5% of pollen and 5% of other substances considering organic rubbles. In some propolis samples, more than 180 constituents have been identified.

The use of propolis has been known since 300 bc, and many biotherapeutic uses have been reported since then, such as, anticancerous, antioxidant, antinflamatory, antifungal, antihepatotoxic, in addition to its antibacterial effect that is of interest in this invention, because may act against microorganisms associated to cavities. Although propolis may have a big potential controlling bacteria associated to cavities as S. *mutans,* there is not much information related to this respect.

The more important propolis constituents pharmacologically active are: flavonoids (flavones, flavonols, flavonons), phenolic compounds and aromatics. Flavonoids are related to most of the biological activity of propolis. The antibacterial activity is attributed to a flavonon pinocembrin, flavonol galangin and caffeic acid phenyl ester (CAPE) with a mode of action probably based on the inhibition of the glucosyltransferase bacterial enzyme.

Propolis consistence varies with temperature. At 15°C is found hard and breakable, at 30° C is soft and malleable, from 30 to 60°C is sticky and rubbery and its melting points is at 70 ° C. Propolis is insoluble in water but specially soluble in ethanol, acetone, benzene, ammonia, chloroform, ether, trichloroethylene, etc. Most of products based on propolis may be dissolved using a mixture of al these solvents mentioned above. This mixture of organic extracts is considered when liquid propolis is used, and is this extract the one used as active ingredient for this invention. Bacteria have demonstrated the same sensibility to propolis than to antibiotic, but with the difference that propolis do not cause side effects, and with the advantage that it could be naturally eliminated without alteration of the intestinal flora or liver malfunction.

Propolis is non-toxic and daily doses of 1400 mg/kg do not cause negative effects in rats, although there are some reports of beekeepers showing allergic reactions. Propolis is also a natural source of antioxidants that protect to oils and ceric lipoproteins from oxidation. Its antioxidants properties are due to its antiradical activity (alkoxy radicals and in some degree superoxide), and to the inhibitor effect on the cuprous ion, which is an indicator of low density proteins oxidation.

All these characteristics make to the propolis extract an active ingredient highly desirable in a dentifrice composition. The composition of this invention, which combines the propolis' extracts with a small amount of methylparaben and propilparaben reinforces the antibacterial effect produced by the propolis extracts. As mentioned before, the dentifrice composition of this invention has 2 to 10% of extract of propolis, 0,01 to 0,30% of methylparaben and 0,01 to 0,15% of propylparaben. In a preferred formulation the composition of this invention has 4 to 8 % of propolis extract, 0,10 to 0,20% of methyilparaben, and 0,05 to 0,10% of propylparaben.

The composition also comprises a fluorinated compound as active ingredient, where this compound is found in a percentage between 0,2 to 2% with respect to the total weight of the dentifrice composition. A preferred composition of the invention has between 0,4 to 1,6 active fluorinated compounds. Where this active fluorinated compound is chosen among the following compounds and their combinations: sodium fluoride, sodium monofluorphosphate, nicomethanol fluorhydrate (fluorinol), stannous fluoride, amine fluoride or potassium fluoride. The dentifrice composition of the invention also considers 20 to 50% of abrasive compounds. These abrasives are chosen from the following compounds and their combinations: calcium carbonate, amorphous silicon hydride (Aerosil), aluminium hydroxide, calcium monohydrogen phosphate and insoluble sodium metaphosphate.

The anticariogenic dentifrice composition of this invention also has between 20 to 50% of moisturizing ingredients, which are chosen from the following ingredients and their combinations: glycerin, sorbitol solution, polyethylene glycol and propylene glycol. The anticariogenic dentifrice composition of this invention also has between 0,5 to 3% of foam forming compounds. These compounds where chosen from the following ingredients and their combinations: sodium lauryl sulfate, sodium lauryl sarcosinate, sucrose fatty acid ester, *polyoxyethylene hardening castor oil,* polyoxythylene copolymer and polyoxypropylene, carrageenan, xanthan gum and sodium alginate. The cariogenic dentifrice composition of this invention also has between 0.05 to 5% of sweetener compounds. These compounds are chosen between the following compounds and their combinations: sodium saccharin, aspartame, sucralose, stevioside, xylitol and glycyrrhizic acid

The anticariogenic dentifrice composition of this invention also has between 0,25 to 15% of flavoring compounds. These flavoring compounds are chosen from the following compounds and their combinations: peppermint oil or peppermint essence, menthol and anethole.

The anticariogenic dentifrice composition of this invention also has between 0 to 1,5% of whitening ingredients, where these whitening compounds were chosen among the following compounds and combinations: titanium dioxide, hydrogen peroxide and sodium bicarbonate. The anticariogenic dentifrice composition of this invention also has between 30 to 60% of demineralized water.

### Examples

### 1. Formulation example.

A toothpaste sample of 1 % propolis was prepared in the Department of Research, Development and Innovation (Knop Laboratory), from which the quality specifications were established. The formulation of this product is (Cont. per 100 g):
27 g of Calcium carbonate (Precipitate);
26 g of Sorbitol (70%);
2 g of Glycerin;
0,2 g of Sucralose;
1,5 g of Amorphous Silicic Anhydride (Aerosil);
2,5 g of sodium alginate
1,2 g of sodium lauryl sulfate 90%;
0,15 g of Methylparaben;
0,075 g of Propylparaben;
0,8 g of Sodium Mono-fluorophosphate;
0,75 g of Essence (peppermint-toothpaste);
6 g of Propolis extract 50% PG;
43,0 g of purified water (Demineralized).

### 2. Comparative example of the antibacterial activity of 1% propolis toothpaste.

The antibacterial activity of the 1% propolis toothpaste (shown in example 1) was compared with other three commercial toothpastes at the Laboratory of Molecular biology and Pharmacogenetics (Universidad de la Frontera). The commercial toothpaste evaluated were:
- *Colgate Total 12 Clean Mint,* which has Triclosan (positive control)
- *Colgate Herbal whitening*
- *Sorriso Herbal Gel,* which has propolis

The toothpaste **Colgate Total 12** was considered as **positive control,** which has as active ingredients **Sodium fluoride** (1450 ppm de Flúor) and 0,3% **Triclosan.** Triclosan (5-chloro-2-(2,4-dichlorophenoxy) phenol), which as was mentioned before in the background of the invention, has a powerful antimicrobial activity on bacteria and fungi of the oral cavity.

Three assays were conducted to evaluate the antibacterial activity of:
1.- Toothpaste aqueous extract;
2.- Ethanolic extract toothpaste; and
3.- Complete toothpaste.

### 2.1 Preparation of toothpaste extracts

For extracts preparation a toothpaste dilution was made in a volume of ethanol (70%) and distilled water in a concentration of 1:10, respectively. Then, the extract was used for:
a) to determine the concentration of total polyphenols and,
b) to dilute 1:4 (50 µL + 150 µL) in tripticase soy broth medium (CST) for the antibacterial assay against *Streptococcus mutans* strain, which was previously confirmed by molecular methods.

### 2.2 Determination of polyphenols from toothpaste extracts

The method Folin-Ciaocalteu was used to determine the total polyphenols present in extracts evaluated. Briefly, from each extract, 40 µl were mixed with 560 µl of distilled water, 100 µl of Folin-Ciocalteu reactive (Merck, Germany) and 300 µl of sodium carbonate (7,5% p/v). The absorbance was measured at 760 nm after 2 hours of incubation at room temperature. The concentrations were calculated from a calibration curve and expressed in mg/mL equivalent to the combination of the standars pinocembrin/galangin in proportion 2:1.

The results obtained for the determination of total phenols are shown in Table 1. How is observed in Table 1 the toothpaste knop has a higher concentration of total phenols than the other toothpaste evaluated. It is interesting the phenol detection in the toothpaste Colgate Total 12, which is probably due to the presence of triclosan, an aromatic chlorate compound, which has functional groups representative from esters and phenols. Moreover, it is important to mention, that this compound is soluble in water and ethanol and therefore is present in the extracts.

**Tabla 1. Total phenol concentration expressed as equivalent of pinocembrin/galangin (2:1).**

| **Toothpaste** | **Aqueous extract (mg/mL)** | **Ethanolic extract (mg/mL)** |
|---|---|---|
| Knop with propolis | 1,10 | 1,10 |
| Herbal Colgate | 0,20 | 0,20 |
| Sorriso Herbal Gel | 0,03 | 0,02 |
| Colgate Total 12 (Control +) | 0,20 | 0,20 |

### 2.3 In vitro evaluation of the toothpaste antibacterial activity

The antibacterial activity assay was conducted for each extract through successive dilutions in 96 wells plate. For this, 50 µL of extract were added to 150 µL in tripticase soy broth medium (CST). From this dilution 100µL were taken and diluted in 100 µL of CST. This process was repeated 7 times. After that, 100 µL were inoculated to the suspension of *Streptococcus mutans* to each well, obtaining the following dilution from the extract, either aqueous or ethanolic:

| **Final dilution** | | | | | | | |
|---|---|---|---|---|---|---|---|
| 1/80 | 1/160 | 1/320 | 1/640 | 1/1280 | 1/2560 | 1/5120 | 1/10240 |

In the case of the complete toothpaste, a solution of 10 mg of toothpaste was prepared in a milliliter of the medium CST, and then, the following scheme of diluted was performed: 100 µL of diluted toothpaste was added to 100 µL of tripticase soy broth medium (CST). From this combination100 µL were taken and diluted again in 100 µL de CST. This process was repeated 11 times.

Finally, 100 µL of *Streptococcus mutans* suspension were inoculated to each well obtaining the following concentrations on each well.

| **Final concentration (mg/mL)** | | | | | | | | | | | |
|---|---|---|---|---|---|---|---|---|---|---|---|
| 2,5 | 1,25 | 0,625 | 0,3125 | 0,156 | 0,078 | 0,039 | 0,019 | 0,009 | 0,004 | 0,002 | 0,001 |

The results of the minimum inhibitory dilution (MID) and the minimum inhibitory concentration (MIC) are summarized in Table 2.

With relation to the evaluation of the *In vitro* antibacterial activity of the aqueous extracts, ethanolic extracts and complete toothpaste, it was observed that the minimum inhibitory dilution of the aqueous extracts of the toothpaste: Colgate Herbal, Sorriso Herbal Gel and Colgate Total 12 were similar and better in one dilution than the knop toothpaste. When the minimum inhibitory dilution of the ethanolic extracts were analyzed, it was observed that the minimum inhibitory dilution from the extracts of Colgate Herbal, Sorriso Herbal Gel and Knop were similar (1,25 mg/ml), and that the MIC from the positive control was lower (0,62 mg/ml).

**Table 2. Values of MID and MIC obtained from the antibacterial activity assays conducted in toothpaste.**

| **Toothpaste** | **MID Ex. aqueous** | **MID Ex. ethanolico** | **MIC toothpaste** |
|---|---|---|---|
| Knop with propolis | 1/1280 | 1/1280 | 1,250 mg/mL |
| Colgate Herbal | 1/2560 | 1/1280 | 1,250 mg/mL |
| Sorriso Herbal Gel | 1/2560 | 1/1280 | 1,250 mg/mL |
| Colgate Total 12 (Control +) | 1/2560 | 1/2560 | 0,625 mg/mL |

Although the toothpaste Colgate12 with triclosan has higher antibacterial activity, it is important to mention than the *Environmental Protection Agency* (EPA, USA), still has it registered as pesticide, considering as a risk compound for the environment because its similar composition with other toxic chemical substances. According to the results obtained from some not conclusive studies, EPA will reevaluate the use of Triclosan in 2013, which is ten years ahead schedule. In this regard, there are not drawbacks with toothpaste based on phytotherapics, which makes of this point an important comparative advantage.

These examples demonstrate the effectiveness of the anticariogenic dentifrice composition invented, which has been illustrated in a preferred composition. It will be observed that several modification of this preferred invention could be made without depart from the spirit and scope of this invention as is defined in the attached claims.

## Claims

1. Anticariogenic dentifrice composition characterized because has as anticariogenic active ingredient, which is a combination of propolis, extracts, methylparaben and propylparaben. These components are present in the following percentages in relation to the total weight of the dentifrice composition:
a. 2 a 10 % of propolis extracts;
b. 0,01 a 0,30% of methylparaben; and
c. 0,01 a 0,15% of propylparaben;
where the dentifrice composition additionally has a fluorinated compound and auxiliary components usually used in formulations, such as: abrasives, moisturizing, foaming agents, thickeners, sweeteners, flavorants and demineralized water.

2. Anticariogenic dentifrice composition according to the claim 1, CHARACTERIZED because the fluorinated compound is found in a percentage between 0,2 to 2% with respect to the total weight of the dentifrice composition.

3. Anticariogenic dentifrice composition according to the claim 2, CHARACTERIZED because has as fluorinated compound at least one of the following compounds: sodium fluoride, sodium monofluorophosphate, nicomethanol hydrofluoride (fluorinol), stannous fluoride, amine fluoride or potassium fluoride and their mixtures.

4. Anticariogenic dentifrice composition according to the claim 1, CHARACTERIZED because has between 20 to 50% of abrasives, between 20 to 50% of moisturizing, between 0,5 and 3% of foaming agents, between 0,1 y to 4% of thickeners, between 0,05 to 5% of sweeteners, between 0,25 to 1,5% of flavorants, between 0 to 1,5% of whiteners, and between 30 to 60% of demineralized water.
